(19) Europäisches Patentamt
European Patent Office
Office européen des brevets

(11) Publication number: 0 285 421 B1

# (12) EUROPEAN PATENT SPECIFICATION

(45) Date of publication of patent specification: **19.05.93**

(51) Int. Cl.$^5$: **C12P 9/00**, C12P 19/44, C12P 13/04, //C12N9/16

(21) Application number: **88302894.6**

(22) Date of filing: **30.03.88**

## (54) Method of producing phosphatidic acid derivatives.

(30) Priority: **31.03.87 JP 75907/87**

(43) Date of publication of application:
**05.10.88 Bulletin 88/40**

(45) Publication of the grant of the patent:
**19.05.93 Bulletin 93/20**

(84) Designated Contracting States:
**CH DE FR GB IT LI NL SE**

(56) References cited:
**EP-A- 0 125 039**

**CANADIAN JOURNAL OF BIOCHEMISTRY vol. 51, 1973, Ottawa, CA; N.Z. STANACEV et al.: "Enzymatic formation of cardiolipin from phosphatidylglycerol by the transphosphatidylation mechanism catalyzed by phospholipase D", pages 747-753**

**BIOCHEMICAL JOURNAL, vol. 195, 1981, London, UK; N.G. CLARKE et al.: "Formation of bis(phosphatidyl)inositol and phosphatidic acid by phospholipase D action on phosphatidylinositol", pages 521-523**

(73) Proprietor: **The Japanese Research and Development Association for Bioreactor System**
**No. 17-17, Nihonbashi-kodenma-cho**
**Chuo-ku**
**Tokyo(JP)**

(72) Inventor: **Kudo, Satoshi c/o K.K. Yakuruto Honsha**
**1-1-19, Higashishinbashi**
**Minato-ku Tokyo(JP)**
Inventor: **Sawada, Haruji c/o K.K. Yakuruto Honsha**
**1-1-19, Higashishinbashi**
**Minato-ku Tokyo(JP)**
Inventor: **Watanabe, Tsunekazu c/o K.K. Yakuruto Honsha**
**1-1-19, Higashishinbashi**
**Minato-ku Tokyo(JP)**
Inventor: **Kuroda, Akio c/o K.K. Yakuruto Honsha**
**1-1-19, Higashishinbashi**
**Minato-ku Tokyo(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 285 421 B1

(74) Representative: **Brewer, Leonard Stuart et al**
**SANDERSON & CO. European Patent Attorneys 34, East Stockwell Street**
**Colchester Essex CO1 1ST (GB)**

## Description

BACKGROUND OF THE INVENTION

Field of the Invention

The present invention relates generally to a method of producing phosphatidic acid derivatives. Known lecithins such as soybean lecithin and egg yolk lecithin have been widely used in foods, cosmetics, paints, lubricants, magnetic materials, animal feeds, medicine and agricultural chemicals, and new phosphatidic acid derivatives obtained by causing enzymes having transphosphatidylation to act on lecithins and compounds containing hydroxyl groups are useful substances having advantageous properties over the raw material lecithins in many cases. Therefore, the invention is particularly related to improvements in the method of producing phosphatidic acid derivatives from lecithins as raw materials by the transphosphatidylation reaction.

Description of the Prior Art

It is known in the art that while causing phopholipase D to act on a phospholipid or a derivative of phosphatidic acid in the presence of water results in the production of phophatidic acid by hydrolysis, if, in this case, a compound containing an alcoholic hydroxyl group capable of serving as an acceptor for the phosphatidyl residue and divalent metal ions are allowed to coexist, the following transphosphatidylation is caused to produce a new phosphatidic acid derivative.

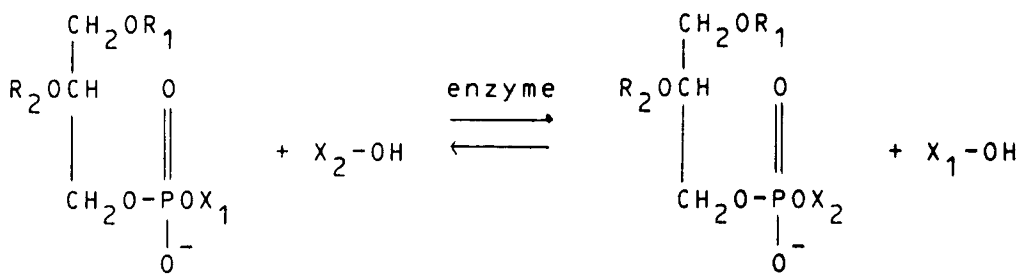


(phospholipid)

(In the formula, $R_1$ and $R_2$ represent acyl groups, alkyl groups or the like, $X_1$ represents a residue remaining after the removal of one hydroxyl group from a sugar or a compound containing alcoholic hydroxyl group and $X_2$ represents a residue remaining after the removal of one hydroxyl group from a compound containing hydroxyl group such as a straight-chain aliphatic polyhydric alcohol or a sugar.)

The divalent metal ion is considered to be essential for causing the phospholipase D to exhibit a transphosphatidylation activity (as an exception, there has been reported (Can.J.Biochem., 51, 747, 1973) that no divalent metal ion is required in the transphosphatidylation reaction between the same kind of phospholipid molecule or phosphatidylglycerol, and therefore it has been the usual practice in the past to infallibly activate the phospholipase D with a divalent metal ion (usually a calcium ion) in the production of phophatidic acid derivative by the ordinary transphosphatidylation reaction between the molecules of different phospholipid (Japanese Laid-Open Patents No. 58-63388, No. 58-67183, No. 59-187792, No. 62-36195, etc.).

Where the calcium ion is used for the activation of the phospholipase D, it is impossible to add to the reaction system any substance which is reactive with this ion and also it is impossible to employ, as a phosphatidyl residue acceptor, any compound containing hydroxyl group which loses its reactivity when combined with the calcium ion. For example, where there is used an enzyme whose most suitable pH for transfer reaction is on the order of 6 to 7, the most usable and inexpensive phosphate buffer cannot be used since it reacts with the calcium ion to cause precipitation, which forces to employ a more expensive buffer solution. Moreover, a part of the final product takes the form of a calcium salt type so that where a product of any other salt type, e.g., sodium salt type is to be produced; it is necessary to perform an additional operation, e.g., the operation of converting the undesired salt to the desired salt by means of a chelating agent or the like.

## SUMMARY OF THE INVENTION

Thus, in view of the fact that the conventional methods of producing phosphatidic acid derivatives through the use of lecithins and phospholipase D suffer various restrictions due to the use of divalent metal ions as mentioned previously, it is the primary object of the present invention to provide an improved production method employing phospholipase D activation process which utilizes no divalent metal ion, thereby making the production of phosphatidic acid derivatives easier than previously.

The method of the invention succeeding in accomplishing the above object has a feature that organic solvent having a phospholipase D activating action is employed for the activation of phospholipase D in place of any divalent metal ion.

In other words, according to one aspect of the invention, in the production of phosphatidic acid derivative by acting phospholipase D on a phospholipid in the presence of a phosphatidyl residue acceptor thereby causing transphosphatidylation reaction, the activation of the phospholipase D is effected by use of organic solvent in the absence of any divalent metal ion to cause the pospholipase D to exhibit a transphosphatidylation activity.

Preferably, the organic solvent for effecting the activation of the phospholipase D is selected from the group consisting of esters having carbon number of 4 to 12, ketones having carbon numbers of 5 to 9 and halogenated hydrocarbons. In this case, it is preferable to use an ethyle acetate as the organic solvent.

In accordance with the method of the invention in which the transphosphatidylation activity of the phospholipase D is caused by the organic solvent without using any divalent metal ion, the entry of any divalent metal ion into the reaction system is prevented so that not only the use of a phosphate buffer is free and a wide range of choice is allowed for the phospholipase D and the reaction conditions to easily effect the reaction under the optimum conditions, but also the separation and purification of the object product from the reaction mixture are simplified. In addition, it is possible to use as reaction raw materials any lecithins and compounds containing alcoholic hydroxyl group which have heretofore been unusable due to the transfer reaction being impeded directly by divalent metal ions, and therefore the present invention gives a chance for providing new phosphatidic acid derivatives.

## DESCRIPTION OF THE INVENTION

Organic solvents, above all, diethyle ether is frequently used as a constituent component of a reaction system in the conventional transphosphatidylation. In the conventional methods, however, the organic solvent is simply used as a solvent for solving a hardly water soluble phospholipid and bringing it into contact with an enzyme and a phosphatidyl residue acceptor in an aqueous solution, and a divalent metal ion, e.g., calcium ion is infalliably used separately for the activation of the enzyme.

The present invention which effects the activation of an enzyme with an organic solvent without using any divalent metal ion, is based on the following new finding by the inventors.

(a) When a certain kind of organic solvent (e.g., ethyl acetate or 4−heptanone) is added to the reaction system in an amount greater than a certain proportion, phospholipase D is activated even in the absence of any divalent metal ion.

(b) Some other kind of organic solvents (e.g., lower aliphatic primary alcohol, dimethylformamide or dimethyl sulfoxide) does not practically activate the phospholipase D but it rather inactivates.

(c) Even among the solvents capable of activating the phospholipase D, there are many (e.g., carbon tetrachloride, n−hexane, benzene, 2−butanol and dioxane) which inactivate the phospholipase D when the concentration is greater than a certain value.

(d) Also, there are organic solvents which are practically inactive on the phospholipase D, that is, those which neither activate nor inactivate the phospholipase D.

Thus, in accordance with the invention, an organic solvent which acts to cause the phospholipase D to exhibit a transphosphatidylation activity is used under such conditions that the activation action is caused more strongly than inactivation action of the enzyme, thereby activating the phospholipase D.

The followings show organic solvents which when used in proper amounts are capable of activating the phospholipase D and having an excellent ability to dissolve or disperse a phospholipid and hence are suitable for use in the method of the invention.

Esters: Esters from aliphatic carboxylic acids having carbon numbers of 2 to 10 and aliphatic alcohols having the same range of carbon numbers are especially preferred. They include, for instance, ethyl acetate, butyl acetate, amyl acetate, decyl acetate, ethyl propionate, propyl propionate, ethyl butyrate, butyl butyrate, isobutyl isobutyrate, ethyl isovalerate, etc. In particular, ethyl acetate is the most preferred phospholipase D activator for use in the production method of the invention in that not only the

transphosphatidylation reaction is caused at a high rate (greater than about 90%) with the resulting reduction in the secondary production or phosphatidic acid, which has never been possible by the conventional production methods employing divalent metal ions but also it is easy to handle and low in cost. Methyl formate and ethyl formate are somewhat high in enzyme inactivation action and are not well suited for use. Esters obtained from higher aliphatic carboxylic acids or alcohols having carbon numbers of over 11 and esters of hydroxy carboxylic acids (e.g., lactic acid, fumaric acid and citric acid) are low in phospholipase D activation action.

Ketones: Aliphatic ketones having carbon numbers of 5 to 9, such as, 3 – pentanone, methyl isobutyl ketone, 4 – heptanone and di – isobutyl ketone are particularly preferred. Those having carbon numbers of less than 5, e.g., propanone and butanone are high in enzyme inactivation action.

Halogenated hydrocarbons: Dichloromethane, trichloromethane, dichloroethane, etc., are preferred.

These preferred solvents, on the other hand, have a lisk of inactivating the enzyme depending on their addition amounts and an activating concentration varies considerably with respective solvents thus causing the solvents to differ in suitable addition amount from each other.
In many case, however, the suitable addition amount of the organic solvent is in the range from 0.05 to 50 parts by volume per 1 part by volume of water. The most desirable amounts of the respective organic solvents can be easily determined by experiments.

In accordance with the production method of this invention, any phospholipid or "phosphatidyl residue donor" can be used as the raw material which belongs to a glycerophosphatide. For example, it is possible to use such synthetic substances identical to phosphatidylcholine and phosphatidylethanolamine in addition to soybean lecithin and egg yolk lecithin.

A suitable compound for use as another reaction raw material or "phosphatidyl residue acceptor" can be selected as desired from the following compounds containing hydroxyl group, e.g., alcohols: methanol, ethanol, propanol, 2 – propanol, butanol, isoamyl alcohol, hexanol, heptanol, octanol, decanol, glycerine, ethylene glycol, etc.; sugars: glucose, xylose, arabinose, fructose, $\beta$ – D – glucose, mannose, sucrose, lactose, cellobiose, melibiose, etc.; amino – sugars: glucosamine, galactosamine, acetyl galactosamine, etc.; glycosides: sorbitol, mannitol, etc.; phenols: phenol, nitrophenol, dinitrophenol, etc.; and others (e.g., adenosine).

It is to be noted that the object of the invention is to provide improvements on the reaction method and it does not reside in the provision of any particular phosphatidic acid derivative and thus it is completely free to select for the reaction any combination of a lecithin and a phosphatidyl residue acceptor (any phospholipid that does not cause a transphosphatidylation only when applied to the method according to the invention has not yet been found).

There are two kinds of phospholipases D, that is, those extracted from plants such as cabbage, carrot and peanut and others produced from microorganisms and either of the two can be employed for the invention. However, the phospholipase D derived from the microorganisms, particularly actinomycetes are preferred since they are excellent in thermal stability and high in activity. In accordance with the production method of the invention, the phospholipase D can be used by dissolving it into the reaction system or in the form of an immobilized enzyme.

Where a lecithin, a compound serving as a phosphatidyl residue acceptor, phospholipase D and organic solvent serving as an activator for the phospholipase D are mixed to cause transphosphatidylation reaction, there is no particular limitation to the order in which they are mixed. However, the followings show the typical methods.

A. The lecithin is dispersed and suspended in water and then the compound serving as a phosphatidyl residue acceptor, the phospholipase D solution and the organic solvent serving as an enzyme activator are added, agitated and mixed.

B. The lecithin is dissovled into the organic solvent serving as an enzyme activator and then mixed with an aqueous solution of the compound serving as a phosphatidyl residue acceptor and the phospholipase D.

C. The lecithin and the compound serving as a phosphatidyl residue acceptor are dissolved into the organic solvent serving as an enzyme activator and then mixed with an aqueous solution of the phospholipase D.

If necessary, any other desired auxiliary constituents such as a buffer for pH adjusting purposes and a surface active agent for dispersing the phospholipid or its solution may be added to a reaction system in such amounts which do not impede the enzyme action. However, the invention does not employ any divalent metal ion, although it does not impede the activation of the phospholipase D by the organic solvent.

Where the phospholipase D is in the form of an immobilized enzyme, the reaction system should preferably be converted to a stable homogeneous phase requiring no agitation. In this case, it is desirable to

contact the immobilized enzyme with a transparent homogeneous phase formed when the reaction system constituents are combined in specific proportions. The transparent homogeneous phase is formed by virtue of the surface activity of the phospholipid when the phospholipid is dissolved to a relatively high concentration in the enzyme activating organic solvent and then a solution of a compound serving as a phosphatidyl residue acceptor is added within certain limits and agitated. There are instances where the formation of the homogeneous phase is facilitated by the addition of a small amount of a hydrophilic organic solvent other than the organic solvent serving as an enzyme activator. For instance, a mixture having the composition of egg yolk lecithin 20 % by weight, glycerin 5 % by weight, ethyl acetate 60 % by weight, 2-propanol 5 % by weight and water 10 % by weight can easily form a homogeneous phase.

After the reaction has been effected sufficiently at around the optimum temperature for the enzyme used, the desired phosphatidic acid derivative can be separated from the reaction mixture by the solvent separation method employing acetone or ethanol, the column chromatography on a silica gel, alumina or reverse-phase carrier, the thin-layer chromatography or the like.

## EXAMPLES

The present invention will now be described in greater detail by way of the following examples.

### Example 1

100 µl of glycerin serving as a phosphatidyl residue acceptor, 400 µl of phosphate buffer having a pH of 6.0 and one unit of actinomyces-derived phospholipase D are added to 5 mg of phosphatidylcholine prepared from an egg yolk lecithin and they are mixed.

After 0 to 100 µl of ethyl acetate has been added to the thus obtained mixture, the amount of phosphatidylglycerol produced by transphosphatidylation reaction is determined with time while shaking the mixture at 50 °C. The results are shown in the following Table 1 showing that when the added amount of ethyl acetate is over 30 µl, the production of phosphatidylglycerol is effected even no divalent metal ion is added.

Table 1 Production Rate of Phosphatidylglycerol

| Ethyl acetate addition (µl) | Production rate (µg/unit min) |
|---|---|
| 0 | 0 |
| 10 | 0 |
| 30 | 35 |
| 50 | 124 |
| 75 | 143 |
| 100 | 101 |

### Example 2

2.5 g of soybean lecithin having a phosphatidylcholin content of about 90 %, 100 ml of 30 % (w/v) phosphatidyl residue acceptor solution , 2 ml of actinomyces-derived phospholipase D solution and 375 ml of ethyl acetate are mixed and agitated to react at 50 °C for 60 minutes.

The following Table 2 shows the results obtained by performing the above method by using different acceptors. Note that in the Table the percentages represent the relative weight percentages to the whole phosphatidic acid derivative including the unreacted lecithin in the reaction mixture.

Table 2

| Acceptor | Transfer Reaction Product (%) |
|---|---|
| Xylose | 72.2 |
| Galactose | 81.3 |
| Amygdaline | 8.6 |
| Sorbitol | 74.2 |
| Serine | 46.5 |
| Glycerin | 83.8 |

Example 3

2.5 g of soybean lecithin having a phosphatidylcholine content of about 90 %, 30 ml of glycerin serving as a phosphatidyl residue acceptor, 40 ml of buffer solution (pH: 5.5), 2 ml of actinomyces-derived phospholipase D solution and organic solvent serving as a phospholipase activating reagent are mixed and reacted at 50 °C for 20 minutes.

The above production method was performed by employing different kinds of organic solvents and different addition amounts and the yields of phosphatidylglycerol or the products of transphosphatidylation were examined. As a result, when organic solvents of the following group A were used, the yields of greater than 20 % could be attained at any addition amount within the range of the experimental levels (the organic solvent addition amounts of 10 to 400 ml). Also, the yields of 10 to 20 % were attained in the case of the compounds of the group B.

A: isopropylether, 2-pentanone, 3-pentanone, methylisobutylketone, 4-heptanone, diisobutylketone, methyl formate, ethyl formate, ethyl acetate, n-propyl acetate, isopropyl acetate, n-butyl acetate, isobutyl acetate, sec-butyl acetate, n-amyl acetate, isoamyl acetate, n-decyl acetate, ethyl propionate, ethyl butyrate, ethyl isobutyrate, ethyl valerate, ethyl isovalerate, n-propyl propionate, n-butyl butyrate, 2-decanol, benzene, toluene, xylene, dichloroethane and phenylmercaptane.

B: ethylether, butylether, tetrahydrofuran, dioxane, acetylacetone, ethyl octanonate, diethyl fumarate, diethyl maleate, diethyl succinate, triethyl tartrate, isoamyl isovalerate, 2-propanol, 2-butanol, heptanol, 2-heptanol, octanol, pentane, hexane, heptane, isooctane, cyclohexane, dichloromethane, trichloromethane and tetrachloromethane.

## Claims

1. A method of producing a phosphatidic acid derivative by acting phospholipase D on a phospholipid in the presence of a phosphatidyl residue acceptor to cause transphosphatidylation reaction, characterized by the step of activating said phospholipase D by organic solvent in the absence of divalent metal ion to cause said phospholipase D to exhibit a transphosphatidylation activity.

2. A method according to claim 1, wherein said phospholipase D is activated by organic solvent selected from a group consisting of esters having carbon numbers of 4 to 12, ketones having carbon numbers of 5 to 9 or halogenated hydrocarbons.

3. A method according to claim 2, wherein ethyl acetate is used as said organic solvent.

## Patentansprüche

1. Verfahren zur Herstellung eines Phosphatidsäurederivates durch Einwirkung von Phospholipase D auf ein Phospholipid in Anwesenheit eines Phosphatidylrestakzeptors unter Bewirken einer Transphosphatidylierungsreaktion, gekennzeichnet durch den Schritt der Aktivierung der Phospholipase D durch ein organisches Lösungsmittel in Abwesenheit eines zweiwertigen Metallions, um die Phospholipase D zu veranlassen, eine Transphosphatidylierungsreaktion zu entfalten.

**2.** Verfahren gemäß Anspruch 1, bei welchem die Phospholipase D durch ein aus einer aus Estern mit Kohlenstoffzahlen von 4 bis 12, Ketonen mit Kohlenstoffzahlen von 5 bis 9 oder halogenierten Kohlenwasserstoffen bestehenden Gruppe ausgewähltes Lösungsmittel aktiviert wird.

**3.** Verfahren gemäß Anspruch 2, bei welchem Essigsäureethylester als organisches Lösungsmittel verwendet wird.

## Revendications

**1.** Procédé de production d'un dérivé d'acide phosphatidique en faisant agir la phospholipase D sur un phospholipide en présence d'un accepteur de reste de phosphatidyle afin de provoquer une réaction de transphosphatidylation, caractérisé en ce qu'on active ladite phospholipase D par un solvant organique en l'absence d'un ion de métal divalent afin d'obliger ladite phospholipase D à faire preuve d'une activité de transphosphatidylation.

**2.** Procédé selon la revendication 1, dans lequel ladite phospholipase D est activée par un solvant organique choisi parmi les esters en $C_{4-12}$, les cétones en $C_{5-9}$ et les hydrocarbures halogénés.

**3.** Procédé selon la revendication 2, dans lequel on utilise de l'acétate d'éthyle en qualité dudit solvant organique.